# FASCICULE DE BREVET EUROPEEN

(11) **EP 0 801 744 B1**
(45) Date de publication et mention de la délivrance du brevet: **02.05.2002**
(21) Numéro de dépôt: 96901017.2
(22) Date de dépôt: 04.01.1996
(51) Int. Cl.: G01N 33/569

(54) **REACTIF PEPTIDIQUE PERMETTANT DE DETECTER UNE INFECTION PRIMAIRE A VIRUS D'EPSTEIN-BARR PAR RECHERCHE DES ANTICORPS CORRESPONDANTS, ET PROCEDE D'UTILISATION DE CE REACTIF**
PEPTIDISCHES REAGENZ UND VERFAHREN ZUM NACHWEIS EINER PRIMÄRINFEKTION DURCH DEN EPSTEIN-BARR-VIRUS MITTELS TEST DER ENTSPRECHENDEN ANTIKÖRPER
PEPTIDE REAGENT FOR DETECTING A PRIMARY EPSTEIN-BARR VIRUS INFECTION BY TESTING FOR THE CORRESPONDING ANTIBODIES, AND METHOD FOR USING SAME

(30) Priorité: 04.01.1995 FR 9500036
(43) Date de publication de la demande: 22.10.1997
(73) Titulaire: INSTITUT PASTEUR DE LYON, F-69007 Lyon (FR); PARTEUROP DEVELOPPEMENT, 69007 Lyon (FR)
(72) Inventeur: DROUET, Emmanuel, F-38700 Corenc (FR); BREBANT, Richard, F-69280 Marcy-l'Etoile (FR)
(74) Mandataire: Tonnellier, Jean-Claude
(86) Numéro de dépôt international: FR9600014
(87) Numéro de publication internationale: WO9621155

(56) Documents cités:
- EP-A- 0 154 917
- WO-A-92/00525
- RESEARCH IN VIROLOGY, vol. 144, no. 5, 1993 pages 397-404, V. MARECHAL ET AL. 'Enzyme-linked immunosorbent assay to ZEBRA, and Epstein-Barr trans-activator.'
- JOURNAL OF CLINICAL MICROBIOLOGY, vol. 29, no. 10, Octobre 1991 pages 2180-2186, H-M CHENG ET AL. 'Epstein-Barr virus nuclear antigen 1 linear epitopes that are reactive with immunoglobulin A (IgA) or IgG in sera from nasopharyngeal carcinoma patients or from healthy donors.'
- JOURNAL OF IMMUNOLOGICAL METHODS, vol. 182, 9 Juin 1995 pages 227-234, NIVELEAU ET AL. 'Grafting peptides onto polystyrene microplates for ELISA'
- CANCER IMMUNOL. IMMUNOTHER., vol. 40, 1995 pages 251-256, H-M. CHENG ET AL. 'Linear epitopes of the replication-activator protein of Epstein-Barr virus recoganised by specific serum Ig G in nasopharyngeal carcinoma.'
- JOURNAL OF GENERAL VIROLOGY, vol. 76, 1995 pages 1393-1400, R. TEDESCHI ET AL. 'The disease associations of the antibody response against the Epstein-Barr virus transactivator protein ZEBRA can be separated into different epitopes.'
- Brousset et al., AIDS, 1994, Vol 8 No 5, pp. 583-590; Joab et al., Int.J.Cancer, 1991, 48, pp. 647-649

## Description

L'invention a pour objet l'utilisation d'un réactif peptidique permettant la détection, même à un stade très précoce, d'une infection primaire par le virus d'Epstein-Barr, par recherche dans un échantillon biologique de la présence éventuelle d'anticorps IgM reconnaissant ledit réactif. L'invention concerne également un procédé de détection d'une infection primaire par le virus d'Epstein-Barr à l'aide d'un tel réactif peptidique, ainsi qu'un nécessaire (trousse de détection) permettant la mise en oeuvre de ce procédé.

On sait que le virus d'Epstein-Barr (en abrégé : EBV) est un virus capable d'infecter les cellules épithéliales et lymphoïdes humaines. Il est établi que ce virus est à l'origine de la mononucléose infectieuse (en abrégé : MNI). La primo-infection survient le plus souvent pendant l'enfance, généralement de façon asymptomatique, et le virus reste ensuite présent dans l'organisme, à l'état latent. On estime généralement que plus de 95% des humains adultes sont infectés par ce virus. Dans certains pays et/ou dans certains groupes sociaux, l'infection primaire peut n'avoir lieu qu'à l'âge de l'adolescence ou chez l'adulte jeune. Parmi ces cas d'infections primaires tardives, 50% environ sont des infections aiguës accompagnées des symptômes de la mononucléose infectieuse (MNI).

On sait par ailleurs que dans certaines zones géographiques, l'EBV est étroitement associé avec certains cancers, notamment le cancer du nasopharynx et le lymphome de Burkitt.

Chez les personnes immunodéprimées, et notamment les personnes ayant subi des greffes d'organes ainsi que chez les patients infectés par le virus HIV, on observe fréquemment une « réactivation » du virus, c'est-à-dire le passage d'un état de latence à un cycle lytique de réplication du virus. Il est connu que chez les sujets immunodéprimés, la réactivation du virus ou l'infection primaire conduit fréquemment au développement de divers lymphomes.

L'étude de l'infection à EBV et la recherche de méthodes de diagnostic sérologique a permis de mettre en évidence plusieurs antigènes viraux : les antigènes de capsides virales (VCA), les antigènes précoces (EA) et les antigènes nucléaires (EBNA).

Le diagnostic sérologique classique des infections à EBV comprend d'une part la recherche d'anticorps hétérophiles (test de Paul-Bunnell-Davidsohn) et la recherche d'anticorps contre les antigènes VCA, EA et EBNA, généralement par immunofluorescence indirecte. Ces tests sont difficiles à mettre en oeuvre et on observe une certaine proportion de faux négatifs et de faux positifs.

On admet actuellement que les profils sérologiques peuvent être interprétés de façon non équivoque dans certains cas qui sont passés en revue ci-après.

L'apparition d'anticorps anti-VCA suit généralement de près la primo-infection et on admet que l'absence d'IgG anti-VCA permet de conclure que le sujet n'a pas été infecté par l'EBV. On admet d'ailleurs que lorsqu'un sérum ne contient pas d'anti-VCA, il ne contient pas non plus d'autres anticorps anti-EBV

Chez les individus porteurs asymptomatiques, c'est-à-dire chez la presque totalité de la population qui a été infectée par le virus et chez qui le virus est à l'état de latence, on trouve à la fois des anticorps anti-VCA et anti-EBNA.

Dans le cas de l'infection primaire symptomatique (MNI), la recherche d'anticorps hétérophiles (Test de Paul-Bunnell-Davidsohn, ou PBD) est généralement positive. On trouve généralement la présence d'IgG anti-VCA tandis que les anticorps anti-EBNA sont absents ou ne sont présents qu'en faible quantité. Les IgG anti-VCA diminuent progressivement après l'infection primaire et restent stables toute la vie, tandis que les anticorps anti-EBNA apparaissent plus tardivement, au bout de plusieurs mois avant de se stabiliser.

A propos de l'état des connaissances sur les infections à EBV et leur diagnostic, on peut citer notamment J.-M. Seigneurin - Infections à Virus Epstein-Barr - Editions techniques - Encycl. Méd. Chir. (Paris-France), Maladies Infectieuses, 8-071-A-10, Pédiatrie 4-310-A-30, pp. 1-7 (1993).

On sait par ailleurs qu'une protéine appelée ZEBRA (ou EB1 ou Zta) joue un rôle primordial dans la régulation de la latence ; voir notamment G. Miller, J. of Infectious Diseases, 161:838-844 (1990) et I. Mikaélian et al., J. Virol., 67:734-742 (1993).

Cette protéine est un facteur de transcription qui joue notamment un rôle d'activateur dans la transcription de certains gènes et dans sa propre synthèse. La protéine ZEBRA est considérée comme étroitement associée à la transition entre l'état de latence et le cycle lytique du virus, et la recherche d'anticorps anti-ZEBRA a été étudiée comme un marqueur indirect d'une réactivation virale, notamment chez des sujets immunodéprimés HIV-séropositifs ; voir par exemple I. Joab et al., J. of Infectious Diseases, 163:53-56 (1991) et V. Maréchal et al., Res. Virol., 144:397-404 (1993).

L'article de Joab et *al*., ainsi que l'article de Brousset et *al*., AIDS 8 (5), 583-590 (1994) mentionnent la présence d'anticorps anti-ZEBRA ou anti-peptides de ZEBRA dans des cas de MNI.

On a maintenant découvert que les anticorps anti-ZEBRA apparaissent très précocement lors des infections primaires, et que la détection de la présence d'anticorps IgM anti-ZEBRA permet de diagnostiquer de façon très précoce les primo-infections à EBV. On a découvert en outre que la recherche d'IgM dirigées contre le fragment 157-195 de la protéine ZEBRA permet de diagnostiquer sans faux négatifs les infections primaires à un stade où aucun des marqueurs sérologiques classiques ne donne 100% de résultats positifs.

On rappelle que la séquence 157-195 de la protéine Zebra est un peptide répondant à la formule (I):

Arg Arg Thr Arg Lys Pro Gln Gln Pro Glu Ser Leu Glu Glu Cys Asp
Ser Glu Leu Glu Ile Lys Arg Tyr Lys Asn Arg Val Ala Ser Arg Lys
Cys Arg Ala Lys Phe Lys Gln.

Par une étude systématique de recherche d'anticorps dirigés contre divers peptides plus courts que le peptide de formule (I) et dont la séquence est comprise dans la séquence représentée par la formule (I), on a remarqué que l'on détecte plus particulièrement, dans les infections primaires à EBV, des IgM dirigées contre une partie de l'extrémité carboxy-terminale de la séquence de formule (I). La séquence de cette partie est représentée par la formule (II) suivante :

Leu Glu Ile Lys Arg Tyr Lys Asn Arg Val Ala Ser Arg Lys Cys Arg
Ala Lys Phe Lys Gln.

La présente invention a donc pour objet l'utilisation comme réactif peptidique permettant la détection, et notamment la détection très précoce, d'une infection primaire par le virus d'Epstein-Barr chez un sujet, par recherche, selon une méthode fondée sur la formation d'au moins un complexe du type antigène-anticorps, dans un échantillon biologique prélevé chez ledit sujet, d'anticorps IgM reconnaissant ledit réactif. Cette utilisation est telle que définie dans les revendications annexées.

Compte tenu de la précocité de l'apparition des IgM dirigées contre le peptide de formule (II), l'utilisation du réactif selon l'invention permet aussi de confirmer, avec moins de risques d'erreur qu'en utilisant les méthodes connues jusqu'à présent, l'absence d'une infection primaire à EBV.

On peut donc utiliser notamment comme réactif peptidique selon l'invention un peptide consistant en tout ou partie de la séquence de formule (II), y compris un peptide dont la séquence est plus courte que celle de formule (II), par exemple un peptide de formule (III): Val Ala Ser Arg Lys Cys Arg Ala Lys Phe Lys Gln, ou encore un peptide dont la séquence est plus longue que celle de formule (II) (par exemple le peptide de formule (I)) ou bien un peptide dont la séquence comprend une partie de l'extrémité N-terminale du peptide II complétée, du côté N-terminal, par une séquence adjacente présente dans la formule (I), par exemple un peptide de séquence :

Ser-Glu Leu Glu Ile Lys Arg Tyr Lys Asn Arg Val Ala Ser Arg Lys
Cys Arg Ala Lys Phe.

L'invention a également pour objet un procédé de détection d'une infection primaire par le virus d'Epstein-Barr chez un sujet, caractérisé par le fait que l'on recherche, de façon connue en soi, dans un échantillon biologique prélevé chez ledit sujet, la présence de certains anticorps IgM tels que définis dans les revendications annexées.

Bien entendu, la présence des anticorps IgM ainsi recherchés permet de conclure à l'existence d'une infection primaire à EBV, et l'absence desdits anticorps permet de conclure à l'absence d'une infection primaire détectable.

Selon un mode de réalisation particulier, le procédé de l'invention est un procédé dans lequel :
- on met en contact un échantillon biologique prélevé chez ledit sujet avec un réactif peptidique, dans des conditions permettant la formation d'un complexe de type antigène-anticorps entre ledit réactif peptidique et des anticorps qui reconnaissent ledit réactif, s'ils sont présents dans ledit échantillon,
- et on recherche la présence éventuelle d'un tel complexe, selon une méthode de révélation connue en soi,
caractérisé par le fait que ledit réactif peptidique consiste en un peptide tel que défini dans les revendications annexées,
et par le fait que les complexes dont on recherche la présence sont des complexes formés entre ledit réactif et des anticorps de type IgM.

Dans les procédés qui viennent d'être décrits, les réactifs peptidiques sont notamment ceux qui ont été définis précédemment.

L'échantillon biologique est notamment un échantillon de sérum sanguin, ou un autre fluide biologique, par exemple le liquide céphalo-rachidien.

La mise en oeuvre des tests de détection d'anticorps à l'aide d'un antigène est bien connue et ne sera donc pas décrite en détail ici. L'antigène peut être fixé, par adsorption ou par covalence, sur un support solide (puits d'une plaque de microtitration, paroi d'un tube, bille de matière plastique, particules de latex, etc). Lorsque l'antigène est fixé sur des microbilles, on peut opérer selon les méthodes d'immunofiltration, et avec des particules de latex, on peut procéder selon des techniques d'agglutination. On peut aussi opérer notamment selon les techniques en phase solide dites « sandwich », ou encore par compétition avec des anticorps marqués spécifiques de l'antigène. Dans le cas des techniques sandwich, la révélation peut être effectuée de façon connue, à l'aide d'anticorps hétérologues (anticorps d'animaux) anti-(IgM humaines) marqués. On peut également utiliser dès anticorps hétérologues anti-(IgM humaines) non marqués et révéler leur fixation sur les IgM, si elles sont présentes, par des anticorps marqués dirigés contre les immunoglobulines de l'espèce animale dont proviennent les anticorps hétérologues utilisés.

Le marquage des anticorps est effectué notamment par couplage avec une enzyme (par exemple une peroxydase) capable de catalyser une réaction colorée. On peut également utiliser un marqueur fluorescent ou luminescent.

On peut aussi mettre en évidence la formation de complexes de type antigène-anticorps sans utilisation d'anticorps marqués, par exemple à l'aide de mesures électriques (capacité, impédance), de façon connue.

L'invention a également pour objet un nécessaire pour la détection d'une infection par le virus d'Epstein-Barr, ledit nécessaire étant tel que défini dans les revendications annexées.

Les exemples suivants illustrent l'invention.

### EXEMPLE 1:

On a effectué des tests de diagnostic en utilisant comme antigène la protéine ZEBRA ou certains peptides contenus dans cette protéine.

On a comparé les résultats à ceux obtenus avec les tests classiques de diagnostic sérologique.

Les tests classiques utilisés sont les suivants :
- recherche d'anticorps hétérophiles (test PBD)
- recherche d'IgG dirigées contre les antigènes VCA, EA et EBNA par immunofluorescence indirecte. Le titrage des anticorps anti-VCA et anti-EA a été réalisé en utilisant les lignées cellulaires P3HR1 et Raji, soumises à une induction (passage du cycle latent au cycle lytique) par le mélange phorbol myristate/butyrate respectivement. Le titrage des anticorps anti-EBNA a été pratiqué en utilisant la lignée Raji (non induite). Les lignées P3HR1 et Raji sont disponibles auprès de nombreux laboratoires et de l'ATCC.

On a utilisé en outre deux kits commerciaux permettant une révélation immuno-enzymatique :
- le test commercialisé sous la dénomination « Biotest anti-EBV recombinant » par Biotest AG (Allemagne) pour rechercher les anticorps anti-EA et anti-EBNA,
- et le test commercialisé sous la dénomination « Enzygnost » par Behring (Allemagne) qui permet une recherche des anticorps IgG et IgM anti-EBV.

Dans tous les cas, les sérums étudiés ont été adsorbés sur le réactif Gull-Sorb (Gull Laboratories, Salt lake City, USA) afin d'éviter les interférences dues au facteur rhumatoïde.

### Définitions

Une infection primaire à EBV est définie par la présence d'IgM anti-EBV et en particulier d'IgM anti-VCA par immunofluorescence indirecte et/ou par la présence d'anticorps hétérophiles. On observe ensuite successivement, au décours de l'infection, la présence d'IgG anti-VCA (> 1:20) et d'IgG anti-EA (> 1:10) avec absence d'anticorps anti-EBNA (< 1:10).

Une réactivation du virus EBV (infection secondaire) est définie par la présence d'anticorps anti-VCA (> 1:320) et anti-EA (> 1:40) avec présence (ou préexistence lorsqu'on a pu l'établir) d'anticorps anti-EBNA (>1:10).

Une infection latente est définie par l'absence d'IgM anti-VCA et l'absence d'IgG anti-EA (< 1:10) avec présence d'IgG anti-VCA (< 1:320) et and-EBNA.

### Peptides utilisés comme antigènes

Le peptide de formule (I) a été synthétisé selon les méthodes classiques. Ce peptide est appelé ici P130.

On a également utilisé comme antigène la protéine ZEBRA clonée dans *Escherichia coli.* Après culture bactérienne, la protéine ZEBRA est extraite et purifiée selon les techniques usuelles.

### Réaction immuno-enzymatique

On revêt les cupules (puits) de plaques de microtitrage avec l'antigène de la façon suivante : le peptide est déposé dans chaque puits à raison de 100 ng/100 µl (dilué en tampon carbonate-bicarbonate 50 mM, pH 9,6) et on laisse incuber pendant une nuit à 37°C.

Les plaques sont ensuite lavées 3 fois avec du tampon phosphate-Tween 0,1% (PBST) pH 7,4. Les sérums sont dilués au 1:100 en tampon PBS (1M NaCl)-sérum de veau foetal 5%-Tween 0,1 % (PBSST). On dépose dans les puits le sérum ainsi dilué à raison de 100 µL/puits. Après incubation pendant 1 heure à 37°C suivie de 3 lavages avec PBST, on ajoute 100 µL de conjugué anti-IgG humaines/peroxydase (ou anti-IgM humaines/peroxydase). Ces conjugués commercialisés par Jackson-Immunoresearch Inc. sont dilués préalablement au 1:26.000 dans du PBST. Après incubation pendant 30 minutes à 37°C, puis lavage avec PBST, on procède à la révélation de l'enzyme pendant 10 minutes à l'abri de la lumière à l'aide d'une solution de tétraméthylbenzidine (0,5%) et de peroxyde d'hydrogène (0,05%) en tampon citrate pH 4 (100 µl/puits). La réaction colorée est stoppée à l'aide d'une solution acide sulfurique 1N. La lecture se fait dans un spectrophotomètre et l'absorbance est mesurée à 450 nm (A₄₅₀).

Chaque dilution de sérum est testée en double, d'une part vis-à-vis de l'antigène (peptide ou protéine ZEBRA purifiée) et d'autre part vis-à-vis de cupules sans antigène (puits témoin). La valeur finale retenue de l'absorbance est donc la valeur résultant de la différence entre l'absorbance moyenne des puits contenant l'antigène et l'absorbance moyenne des puits témoins.

Des sérums de référence positifs (pool de 10 sérums de patients avec MNI) et négatifs (pool de 20 sérums de patients séronégatifs et asymptomatiques) sont également testés en double et dans les mêmes conditions que les échantillons cliniques.

La valeur-seuil de l'absorbance a été fixée à 0,200 pour la méthode Elisa utilisant le peptide de formule (I) et à 0,300 pour la méthode Elisa utilisant la protéine ZEBRA.

20 sérums provenant de patients avec mononucléose infectieuse ont été testés. Les IgM anti-P130 sont détectées dans 100% des sérums et les IgM ariti-ZEBRA dans 55% des cas.

Les IgG anti-P130 et anti-ZEBRA sont détectées dans 75% et 80% des cas, respectivement. Les anticorps IgG anti-VCA, anti-EA et anti-EBNA sont détectés en immunofluorescence dans 100%, 100% et 0% des cas, respectivement.

On a par ailleurs recherché les IgM anti-EA, IgG anti-EA et IgG anti-EBNA avec le test de Biotest. Les résultats sont positifs dans 90%, 80% et 0% des cas, respectivement.

Avec le test ELISA anti-EBV Behring, les IgM et IgG ont été détectées dans 80% et 100% des cas, respectivement.

Dans 6 autres cas étudiés de patients semblant présenter des symptômes de MNI, avec absence d'anticorps anti-VCA, anti-EA et anti-EBNA en immunofluorescence, les IgM anti-EA (test ELISA Biotest) étaient détectées chez 4 patients sur 6, de même que les IgM anti-EBV (Enzygnost), tandis que les IgM anti-P130 étaient détectées dans tous les cas.

Ainsi, sur l'ensemble des sérums testés (MNI ou syndrome MNI-like), seuls les tests effectués avec l'antigène P130 ont permis de détecter une infection primaire et même des infections non déclarées pour lesquelles les tests d'immunofluorescence donnaient des résultats négatifs tandis que les tests commerciaux ELISA ne détectaient pas tous les sérums positifs détectés par P130.

### EXEMPLE 2 : Utilisation de l'antigène de formule (II) ou de formule (III)

Ces peptides ont été synthétisés de façon classique. Avec ces peptides, on a réalisé des tests ELISA de façon analogue à celle décrite à l'exemple 1.

Les résultats sont analogues à ceux observés avec le peptide de formule (I).

## Revendications

1. Utilisation comme réactif peptidique pour la détection d'une infection primaire par le virus d'Epstein-Barr chez un sujet, par recherche, selon une méthode fondée sur la formation d'au moins un complexe du type antigène-anticorps, dans un échantillon biologique prélevé chez ledit sujet, d'anticorps IgM reconnaissant ledit réactif, **caractérisée par le fait que** ledit réactif est un peptide reconnu par au moins un anticorps dirigé contre le peptide dont la séquence est représentée par la formule (II) : 
Leu Glu Ile Lys Arg Tyr Lys Asn Arg Val Ala Ser Arg Lys
Cys Arg Ala Lys Phe Lys Gln,
 et sous réserve que ledit réactif ne soit reconnu que par des anticorps reconnaissant ledit peptide de formule (II).

2. Utilisation selon la revendication 1, **caractérisée par le fait que** la séquence dudit réactif consiste en tout ou partie de la séquence de formule (II).

3. Utilisation selon la revendication 1 ou 2, **caractérisée par le fait que** ledit réactif est reconnu par au moins un anticorps dirigé contre le peptide dont la séquence est représentée par la formule (III) suivante :

Val Ala Ser Arg Lys Cys Arg Ala Lys Phe Lys Gln.

4. Utilisation selon la revendication précédente, **caractérisée par le fait que** la séquence dudit réactif consiste en tout ou partie de la séquence de formule (III).

5. Procédé de détection d'une infection primaire par le virus d'Epstein-Barr chez un sujet, **caractérisé par le fait que** l'on recherche, de façon connue en soi, dans un échantillon biologique prélevé chez ledit sujet, la présence d'anticorps IgM capables de reconnaître le peptide de formule (II):

Leu Glu Ile Lys Arg Tyr Lys Asn Arg Val Ala Ser Arg Lys
Cys Arg Ala Lys Phe Lys Gln.

6. Procédé selon la revendication précédente, dans lequel :
- on met en contact un échantillon biologique prélevé chez ledit sujet avec un réactif peptidique, dans des conditions permettant la formation d'un complexe de type antigène-anticorps entre ledit réactif peptidique et des anticorps qui reconnaissent ledit réactif, s'ils sont présents dans ledit échantillon,
- et on recherche la présence éventuelle d'un tel complexe, selon une méthode de révélation connue en soi,
**caractérisé par le fait que** ledit réactif peptidique est un peptide reconnu par au moins un anticorps dirigé contre le peptide dont la séquence est représentée par la formule (II): 
Leu Glu Ile Lys Arg Tyr Lys Asn Arg Val Ala Ser Arg Lys
Cys Arg Ala Lys Phe Lys Gln,
 et sous réserve que ledit réactif ne soit reconnu que par des anticorps reconnaissant ledit peptide de formule (II),
et **par le fait que** les complexes dont on recherche la présence sont des complexes formés entre ledit réactif et des anticorps de type IgM.

7. Procédé selon la revendication précédente, **caractérisé par le fait que** ledit réactif est tel que défini dans l'une quelconque des revendications 2 à 4.

8. Nécessaire pour la détection d'une infection par le virus d'Epstein-Barr, ledit nécessaire comprenant un réactif peptidique et des moyens pour révéler la présence d'un complexe de type antigène-anticorps, susceptible de se former lorsque l'on met en présence ledit réactif et des anticorps éventuellement présents dans un échantillon biologique à. analyser, **caractérisé par le fait qu'**afin de détecter, y compris à un stade précoce, une infection primaire par ledit virus :
- ledit réactif peptidique est un peptide reconnu par au moins un anticorps dirigé contre le peptide dont la séquence est représentée par la formule (II): 
Leu Glu Ile Lys Arg Tyr Lys Asn Arg Val Ala Ser Arg Lys
Cys Arg Ala Lys Phe Lys Gln,
 et sous réserve que ledit réactif ne soit reconnu que par des anticorps reconnaissant ledit peptide de formule (II),
- et lesdits moyens sont des moyens susceptibles de révéler la présence de complexes de type antigène-anticorps formés par des anticorps de type IgM avec ledit réactif.

9. Nécessaire selon la revendication précédente, **caractérisé par le fait que** ledit réactif est tel que défini dans l'une quelconque des revendications 2 à 4.

## Claims

1. Use as a peptide reagent for detecting a primary Epstein-Barr virus infection in a subject, by testing for IgM antibodies that recognize the said reagent in a biological sample taken from the said subject, according to a method based on the formation of at least one complex of the antigen-antibody type, **characterized in that** the said reagent is a peptide recognized by at least one antibody directed against the peptide whose sequence is represented by the formula (II): 
Leu Glu Ile Lys Arg Tyr Lys Asn Arg Val Ala
Ser Arg Lys Cys Arg Ala Lys Phe Lys Gln,
 and with the proviso that the said reagent is recognized only by antibodies that recognize the said peptide of formula (II).

2. Use according to Claim 1, **characterized in that** the sequence of the said reagent consists of all or part of the sequence of formula (II).

3. Use according to Claim 1 or 2, **characterized in that** the said reagent is recognized by at least one antibody directed against the peptide whose sequence is represented by the following formula (III):

Val Ala Ser Arg Lys Cys Arg Ala Lys Phe Lys
Gln.

4. Use according to the preceding claim, **characterized in that** the sequence of the said reagent consists of all or part of the sequence of formula (III).

5. Method for detecting a primary Epstein-Barr virus infection in a subject, **characterized in that** the presence of IgM antibodies capable of recognizing the peptide of formula (II): 
Leu Glu Ile Lys Arg Tyr Lys Asn Arg Val Ala Ser
Arg Lys Cys Arg Ala Lys Phe Lys Gln
 is tested for, in a manner known per se, in a biological sample taken from the said subject.

6. Method according to the preceding claim, in which:
- a biological sample taken from the said subject is brought into contact with a peptide reagent under conditions permitting the formation of an antigen-antibody type complex between the said peptide reagent and antibodies which recognize the said reagent, if they are present in the said sample,
- and the possible presence of such a complex is tested for according to a visualization method known per se,
**characterized in that** the said peptide reagent is a peptide recognized by at least one antibody directed against the peptide whose sequence is represented by the formula (II): 
Leu Glu Ile Lys Arg Tyr Lys Asn Arg Val Ala Ser
Arg Lys Cys Arg Ala Lys Phe Lys Gln,
 and with the proviso that the said reagent is recognized only by antibodies that recognize the said peptide of formula (II),
and **in that** the complexes whose presence is tested for are complexes formed between the said reagent and IgM type antibodies.

7. Method according to the preceding claim, **characterized in that** the said reagent is as defined in any one of Claims 2 to 4.

8. Kit for detecting an Epstein-Barr virus infection, the said kit comprising a peptide reagent and means for visualizing the presence of an antigen-antibody type complex capable of being formed when the said reagent and antibodies possibly present in a biological sample to be analysed are brought into contact with one another, **characterized in that**, in order to detect, including at an early stage, a primary infection by the said virus:
- the said peptide reagent is a peptide recognized by at least one antibody directed against the peptide whose sequence is represented by the formula (II): 
Leu Glu Ile Lys Arg Tyr Lys Asn Arg Val Ala
Ser Arg Lys Cys Arg Ala Lys Phe Lys Gln,
 and with the proviso that the said reagent is recognized only by antibodies that recognize the said peptide of formula (II),
- and the said means are means capable of visualizing the presence of antigen-antibody type complexes formed by IgM type antibodies with the said reagent.

9. Kit according to the preceding claim, **characterized in that** the said reagent is as defined in any one of Claims 2 to 4.

## Patentansprüche

1. Verwendung als Peptidreagens zur Detektion einer primären Infektion eines Individuums durch den Epstein-Barr-Virus durch Untersuchung nach einem Verfahren, basierend auf der Bildung mindestens eines Komplexes vom Typ Antigen/Antikörper, in einer zuvor von dem Individuum entnommenen biologischen Probe, wobei die IgM-Antikörper das Reagenz erkennen, **dadurch gekennzeichnet, dass** das Reagens ein Peptid ist, welches von mindestens einem gegen das Peptid gerichteten Antikörper erkannt wird, dessen Sequenz von der folgenden Formel (II) dargestellt wird: 
Leu Glu Ile Lys Arg Tyr Lys Asn Arg Val Ala Ser Arg Lys
Cys Arg Ala Lys Phe Lys Gln,
 und mit der Maßgabe, dass das Reagens nur von den Antikörpern erkannt wird, die das Peptid der Formel (II) erkennen.

2. Verwendung gemäß Anspruch 1, **dadurch gekennzeichnet, dass** die Sequenz des Reagens ganz oder teilweise aus der Sequenz der Formel (II) besteht.

3. Verwendung gemäß Anspruch 1 oder 2, **dadurch gekennzeichnet, dass** das Reagens durch mindestens einen Antikörper erkannt wird, der gegen das Peptid gerichtet ist, dessen Sequenz durch die folgende Formel (III) dargestellt wird:

Val Ala Ser Arg Lys Cys Arg Ala Lys Phe Lys Gln.

4. Verwendung gemäß dem vorstehenden Anspruch, **dadurch gekennzeichnet, dass** die Sequenz des Reagenzes ganz oder teilweise aus der Sequenz der Formel (III) besteht.

5. Verfahren zur Detektion einer primären Infektion eines Individuums durch das Epstein-Barr-Virus, **dadurch gekennzeichnet, dass** man auf an sich bekannte Weise in einer zuvor aus dem Individuum entnommenen biologischen Probe die Anwesenheit von IgM-Antikörpern untersucht, die das Peptid der Formel (II) erkennen können:

Leu Glu Ile Lys Arg Tyr Lys Asn Arg Val Ala Ser Arg Lys
Cys Arg Ala Lys Phe Lys Gln

6. Verfahren gemäß dem vorstehenden Anspruch, worin:
- man eine zuvor aus dem besagten Individuum entnommene biologische Probe mit einem Peptidreagens unter Bedingungen in Kontakt bringt, welche die Bildung eines Komplexes vom Typ Antigen/Antikörper zwischen dem Peptidreagens und den Antikörpern, die dieses Reagens erkennen, wenn diese in der besagten Probe vorhanden sind, gestatten,
- und man die mögliche Anwesenheit eines solchen Komplexes nach einem an sich bekannten Bestimmungsverfahren untersucht,
**dadurch gekennzeichnet, dass** das Peptidreagens ein Peptid ist, das durch mindestens einen Antikörper erkannt wird, der gegen das Peptid gerichtet ist, dessen Sequenz durch die Formel (II) dargestellt wird: 
Leu Glu Ile Lys Arg Tyr Lys Asn Arg Val Ala Ser Arg Lys
Cys Arg Ala Lys Phe Lys Gln,
 mit der Maßgabe, dass das Reagens nur durch Antikörpern erkannt wird, die das Peptid der Formel (II) erkennen,
und dadurch, dass die Komplexe, deren Anwesenheit man untersucht, Komplexe sind, die zwischen dem Reagens und Antikörpern vom Typ IgM gebildet werden.

7. Verfahren gemäß dem vorstehenden Anspruch, **dadurch gekennzeichnet, dass** das Reagens wie in einem der Ansprüche 2 bis 4 definiert ist.

8. Kit zur Detektion einer Infektion durch das Epstein-Barr-Virus, wobei der Kit ein Peptidreagens und Mittel zum Bestimmen der Anwesenheit eines Komplexes vom Typ Antigen/Antikörper enthält, der sich bilden kann, wenn man das Reagens und die Antikörper, die gegebenenfalls in der zu analysierenden biologischen Probe vorhanden sind, einander gegenüber bringt, **dadurch gekennzeichnet, dass** man zum Detektieren einer primären Infektion durch dieses Virus, einschließlich eines Frühstadiums:
- das Peptidreagens ein Peptid ist, das von mindestens einem Antikörper erkannt wird, der gegen das Peptid gerichtet ist, dessen Sequenz durch die folgende Formel (II) repräsentiert wird: 
Leu Glu Ile Lys Arg Tyr Lys Asn Arg Val Ala Ser Arg Lys
Cys Arg Ala Lys Phe Lys Gln,
 und mit der Maßgabe, dass das Reagens nur durch diejenigen Antikörper erkannt wird, die das Peptid der Formel (II) erkennen,
- und dass die Mittel Mittel sind, welche die Anwesenheit der Komplexe vom Typ Antigen/Antikörper, gebildet von den Antikörpern des Typs IgM mit dem Reagenzs, aufdecken können.

9. Kit gemäß dem vorstehenden Anspruch, **dadurch gekennzeichnet, dass** das Reagens wie in einem der Ansprüche 2 bis 4 definiert ist.
